# EUROPEAN PATENT SPECIFICATION

(11) **EP 1 809 106 B1**
(45) Date of publication and mention of the grant of the patent: **25.04.2012**
(21) Application number: 05800100.9
(22) Date of filing: 31.10.2005
(51) Int. Cl.: A01N 47/34, A01N 47/40, A01N 51/00

(54) **Use of a mixture of novaluron and imidacloprid oder acetamiprid for controlling insects in crops**
Verwendung einer Mischung aus Novaluron und Imidacloprid oder Acetamiprid zur Insektenbekämpfung in Zuchtpflanzen
Utilisation d'un mélange de novaluron et d'imidacloprid ou d'acetamiprid pour la lutte contre les insectes dans les cultures

(30) Priority: 04.11.2004 IL 16502104
(43) Date of publication of application: 25.07.2007
(60) Divisional application: 10178570.7
(73) Proprietor: MAKHTESHIM CHEMICAL WORKS LIMITED, 84100 Beer Sheva (IL)
(72) Inventor: BARAZANI, Avner, 84965 Omer (IL); BARKAI, Jacob, 79505 Moshav Nir Israel (IL)
(74) Representative: Modiano, Micaela Nadia
(86) International application number: PCT/IL2005/001139
(87) International publication number: WO 2006/048868

(56) References cited:
- WO-A-99/18796
- WO-A-99/18796
- WO-A-99/25188
- WO-A-2005/053394
- WO-A-2005/053394
- DE-A1- 4 409 040
- DE-A1- 4 409 040
- DE-A1- 4 436 268
- DE-A1- 4 436 268
- DE-A1- 19 548 874
- US-A- 6 001 858
- US-A- 6 001 858
- DATABASE CA [Online] CHEMICAL ABSTRACTS SERVICE, COLUMBUS, OHIO, US; MATTOS, MARCO ANTONIO A. ET AL: "Agrochemical strategies evaluation for the control of Bemisia argentifolii Bellows & Perring (Hemiptera: Aleyrodidae) on tomatoes" XP002468799 retrieved from STN Database accession no. 2003:159088 & PESTICIDAS , 12, 131-144 CODEN: PTICEA; ISSN: 0103-7277, 2002,
- DATABASE CA [Online] CHEMICAL ABSTRACTS SERVICE, COLUMBUS, OHIO, US; CHEN, ZHANGYAN ET AL: "Emamectin benzoate-hexaflumuron mixed synergistic insecticide" XP002468800 retrieved from STN Database accession no. 2005:5596 & CN 1 480 040 A (FUJIAN XINNONG DAZHENG BIOENGINEERING CO LTD, PEOP. REP. CHINA FUJIAN) 10 March 2004 (2004-03-10)
- DATABASE CA [Online] CHEMICAL ABSTRACTS SERVICE, COLUMBUS, OHIO, US; MA, YUNSHENG ET AL: "Emamectin benzoate-containing compound insecticide" XP002468801 retrieved from STN Database accession no. 2004:500833 & CN 1 399 884 A (WEN, PEIHONG, PEOP. REP. CHINA; XU, BOYONG; CAO, TONGBO) 5 March 2003 (2003-03-05)
- DATABASE CA [Online] CHEMICAL ABSTRACTS SERVICE, COLUMBUS, OHIO, US; LI, PUCHAO: "Compounded insecticide with benzoylphenylurea pesticide" XP002468802 retrieved from STN Database accession no. 2002:278050 & CN 1 308 861 A (ANYANG FOREST CHEMICALS FACTORY, PEOP. REP. CHINA) 22 August 2001 (2001-08-22)
- DATABASE CA [Online] CHEMICAL ABSTRACTS SERVICE, COLUMBUS, OHIO, US; ZHANG, YESHENG: "Environmentally friendly insecticide for controlling cabbage moth and American fly" XP002468803 retrieved from STN Database accession no. 2001:681653 & CN 1 292 218 A (PEOP. REP. CHINA PEOP. REP. CHINA) 25 April 2001 (2001-04-25)

## Description

### Field of the Invention

The present invention relates to the field of pesticidal compositions, particularly pesticidal composition containing at least two pesticidal active ingredients for improved control of pests.

### Background of the Invention

In the practice of pest control, particularly insect control, there are two main factors which determine the effectiveness of the treatment; 1) immediate action on the pests (known in the art as "knock-down action"), 2) long term action (known also as "residual action"). Effective knock-down insecticides include pyrethroids, organic phosphoric acid esters, neonicotinoids, imidacloprid, acetamiprid and phenyl pyrazoles (fipronil). Effective long term insecticides include insect growth regulators (IGR) of various types, e.g. chitin synthesis inhibitors. Current methods for achieving the desired effect include repeated treatments over time with insecticides which have an effective knock-down action. The disadvantages of repeated treatments are; a) the use of relatively large doses of insecticides over time, which may create environmental problems, and b) there are certain periods within the life cycle of the crop when treatments with effective knock-down insecticides are prohibited since they may be absorbed in the crop or damage the crop. Using long term active insecticides may help in preventing repeated treatments, but may not be effective against certain insects which have reached a certain developmental stage in their life cycle. For example, the group of insecticides known as insect growth regulators (IGR) which generally have effective long term action are almost ineffective against adult insects due to the low knock-down effect.

Combination treatments of knock-down and long term insecticides have been reported. U.S. 6,685,954 reports on the effectiveness of fipronil in combination with IGR of the juvenile hormone mimic group in the treatment of ectoparasites in mammals. However, no publications were found which suggest such a combination for other purposes which are non-veterinary purposes. In other fields of applications such as in the filed of plants and plant matter, issues of phytotoxicity must be considered as well.

WO 99/18796 discloses the use of a combination of a benzylurea compound with a further pesticide such as chloronicotinyl insecticides for controlling pests.

Accordingly, there is a long felt need for an insecticidal composition and method of pest control which will provide effective control of insects by providing effective knock-down and long term action, without creating environmental, toxic, phytotoxic and ecotoxic problems.

It is therefore a purpose of the invention to provide an insecticidal composition which provides effective immediate and long term action against insects.

A further purpose of the present invention is to provide a method for pest control, particularly insect control, which provides effective immediate action and effective long term activity.

Other objects of the invention will become apparent as the description proceeds.

### Summary of the invention

The present invention provides the use of an insecticidal composition comprising (a) at least one insecticidal compound with effective knock-down action selected from among group (A) consisting of imidacloprid and acetamiprid, and (b) novaluron as insecticidal compound with effective long-term action, for insect control in crops and locus thereof. Optionally, said insecticidal composition may further contain excipients and/or additives and/or surface active agents

According to a further aspect of the present invention, there is provided a method for insect control comprising a method for insect control comprising applying at least one insecticidal compound with effective knock-down action selected from the group (A) consisting of imidacloprid and acetamiprid and (b) novaluron as insecticidal compound with effective long-term action to a crop or a locus thereof in need of insect control.

### Detailed Description of a Preferred Embodiment of the Invention

The following description is illustrative of embodiments of the invention. The following description is not to be construed as limiting, it being understood that the skilled person may carry out many obvious variations to the invention.

Throughout the description, percentages of components are by weight, unless specifically noted differently. The terms "benzoyl urea", "benzoylphenyl urea" and "BPU" as used throughout the specification are synonymous.

It has surprisingly been found that the combination of an insecticide with knock-down activity selected from among the groups of pesticides known in the art as neonicotinoids, nitromethylene-neonicotinoids and phenylpyrazoles denoted as group (A) comprising of imidacloprid, acetamiprid, thiamethoxam, thiacloprid, nitenpyram, dinotefuran, clothianidin, abamectin, emamectin, emamectin benzoate and fipronil, with an IGR insecticide selected from among the BPU group (B), provided a synergistic effect and effective insect control.

The long term IGR group of pesticides known as BPU herein designated "group (B)" includes *inter alia,* novaluron, lufenuron, hexaflumuron, triflumuron, diflubenzuron, chlorfluazuron, flufenoxuron, noviflumuron and teflubenzuron. Novaluron is used in the present invention.

"Knock-down" pesticides herein designated "group (A)" include: acetamiprid, imidacloprid, thiamethoxam, thiacloprid, nitenpyram, dinotefuran, clothianidin, abamectin, emamectin, emamectin benzoate and fipronil. Acetamiprid or imidacloprid are used in the present invention.

According to a preferred embodiment of the present invention there is provided the use of an insecticidal composition comprising 0.1% to 60% of at least one knock down insecticide selected from among group (A) consisting of imidacloprid and acetamiprid; and 0.5% to 60% of novaluron as insecticidal compound with long-term action. More preferably said composition contains 10% to 50% of at least one insecticide selected from group (A) and 5 % to 15% of novaluron.

The composition disclosed herein is formulated in a manner which suits the specific application. Non-limiting examples of suitable formulations are; emulsion concentrates (EC), suspension concentrates (SC), water dispersible granules (WDG) and wettable powders (WP) and formulations suitable for oral, topical or injectable veterinary formulations.

According to yet a further embodiment of the compositions disclosed herein, there is provided a synergistic insecticidal composition comprising of at least one knock-down insecticide selected from among group (A) consisting of imidacloprid and acetamiprid; and novaluron as insecticidal compound with long-term action, wherein the weight ratio between the group (A) insecticide and novaluron is in the range of 1:100 to 100:1, more preferably 1:50 to 50:1.

The present invention further provides method for insect control, comprising of applying at least one insecticidal compound with effective knock-down action selected from among group (A) consisting of imidacloprid and acetamiprid; and novaluron as insecticidal compound with effective long-term action to a crop, agricultural product, plant matter, animal or locale. The present method is effective for insect control in agricultural, horticultural, insect control in ornamentals, seed treatment and non-agricultural purposes, e.g. public health, storage and domestic use and veterinary.

According to a preferred embodiment of the method of the present invention, the application of the insecticides is simultaneous, separate or sequential application.

According to a preferred embodiment of the present method, the insecticidal composition is employed for pest control.

According to specific embodiment of the method of the present invention when applied to plant matter, the insecticides of group (A) are applied at a rate of 1g/hectar to 500g/hectar and novaluron is applied at a rate of 1g/hectar to 200g/hectar. Preferably, group (A) insecticides are applied at rate of 100g/hectar to 350g/hectar and novaluron at a rate of 30g/hectar to 100g/hectar.

The present method and composition are particularly effective for controlling insects of, *inter alia,* the orders *Lepidoptera, Coleoptera, Homoptera, Heteroptera, Diptera, Thysanoptera, Orthoptera, Anoplura, Siphonaptera, Mallophaga, Thysanura, Isoptera, Psocoptera* and *Hymenoptera,* as well as representatives of the order *Acarina* of the families *Ixodidae, Argasidae, Tetranychidae* and *Dermanyssidae.*

In addition said method and composition are effective in controlling flies, e.g. Musca domestica, termites, cockroaches and mosquito larvae. The presently claimed method and composition is also suitable for controlling plant-destructive feeding insects in ornamentals and crops of useful plants, especially in cotton (e.g. against *Spodoptera littoralis* and *Heliothis virescens*) and in fruit and vegetables (e.g. against *Laspeyresia pomonella, Cydia pomonella, Lithocolletis blancardella, Stigmella malella, Adoxophyes orana, Psylla piri, Cryptophlebia leucotreta, phyllocnistis citrella, Cydia molesta, Anarsia lineatella, Leptinotarsa decemlineata* and *Epilachna varivestis*), as well as for controlling several species of mites, e.g., *oleivora.*

The present method and composition are further effective for controlling ectoparasites such as *Lucilia sericata,* in domestic animals and productive livestock, e.g. by treating animals, cowsheds, barns, stables, pastures and the like.

According to a particular embodiment of the present method, the pesticidal composition is used to prepare a tank mix which is then applied via spraying to the area, plant matter or crop needing treatment. The active ingredient concentration in the tank mix is adjusted to the particular application, depending on agricultural or non-agricultural application, the crop and the pest. Although various methods of application may be employed as the skilled artisan may appreciate, spraying is the preferred method of application. Various methods of application can also be employed when necessary. For example, in field crops systemic application via an irrigation system can be combined with foliar application.

According to a particular embodiment of the invention, separate application is employed wherein the knock down and long term insecticides are applied separately.

## Claims

1. Use of an insecticidal composition comprising (a) at least one insecticidal compound with effective knock-down action selected from among group (A) consisting of imidacloprid and acetamiprid, and (b) novaluron as insecticidal compound with effective long-term action, for insect control in crops and locus thereof.

2. A use according to claim 1 wherein said insecticidal composition comprises 0.1% to 60% of at least one insecticide from group (A) and 0.5% to 60% of novaluron, preferably 10% to 50% of at least one insecticide from group (A) and 5% to 15% of novaluron.

3. A use according to claim 1 wherein the weight to weight ratio between the group (A) insecticides and novaluron is in the range of 1:100 to 100:1, preferably 1:50 to 50:1.

4. A use according to any of claims 1 to 3 wherein said insecticidal composition further contains excipients and/or additives and/or surface active agents.

5. A method for insect control comprising applying at least one insecticidal compound with effective knock-down action selected from the group (A) consisting of imidacloprid and acetamiprid and (b) novaluron as insecticidal compound with effective long-term action to a crop or a locus thereof in need of insect control.

6. A method according to claim 5 wherein the application of the insecticides is simultaneous, separate or sequential.

7. A method according to claim 5 wherein when applied to crop or field, the insecticides of group (A) are applied at a rate of 1g/hectar to 500g/hectar and novaluron is applied at a rate of 1g/hectar to 200g/hectar, preferably, group (A) insecticides are applied at a rate of 100g/hectar to 350g/hectar and novaluron at a rate of 30g/hectar to 100g/hectar.

## Patentansprüche

1. Verwendung einer Insektizidzusammensetzung, die Folgendes umfasst: (a) mindestens eine Insektizidverbindung mit wirksamer Knockdown-Aktivität, gewählt aus der Gruppe (A) bestehend aus Imidacloprid und Acetamiprid und (b) Novaluron als Insektizidverbindung mit wirksamer Langzeitwirkung, zur Insektenbekämpfung in Zuchtpflanzen und Gebiet davon.

2. Eine Verwendung gemäß Anspruch 1, worin die Insektizidzusammensetzung Folgendes umfasst: 0,1% bis 60% mindestens eines Insektizids aus der Gruppe (A) und 0,5% bis 60% Novaluron, vorzugsweise 10% bis 50% mindestens eines Insektizids aus der Gruppe (A) und 5% bis 15% Novaluron.

3. Eine Verwendung gemäß Anspruch 1, worin das Gewichtsverhältnis zwischen den Insektiziden aus der Gruppe (A) und Novaluron im Verhältnis von 1:100 bis 100:1, vorzugsweise 1:50 bis 50:1, liegt.

4. Eine Verwendung gemäß einem beliebigen der Ansprüche 1 bis 3, worin die Insektizidzusammensetzung weiter Bindemittel und/oder Zusatzstoffe und/oder grenzflächenaktive Stoffe enthält.

5. Ein Verfahren zur Insektenbekämpfung, das Folgendes umfasst: Anwendung mindestens einer Insektizidverbindung mit wirksamer Knockdown-Aktivität, gewählt aus der Gruppe (A) bestehend aus Imidacloprid und Acetamiprid und (b) Novaluron als Insektizidverbindung mit wirksamer Langzeitwirkung auf eine Zuchtpflanze oder ein Gebiet davon, die/das Insektenbekämpfung benötigt.

6. Ein Verfahren gemäß Anspruch 5, worin die Anwendung der Insektizide gleichzeitig, separat oder sequentiell ist.

7. Ein Verfahren gemäß Anspruch 5, worin bei Anwendung auf eine Zuchtpflanze oder ein Feld die Insektizide der Gruppe (A) mit einer Rate von 1g/Hektar bis 500g/Hektar angewandt werden und Novaluron mit einer Rate von 1g/Hektar bis 200g/Hektar angewandt wird; vorzugsweise werden Insektizide der Gruppe (A) mit einer Rate von 100g/Hektar bis 350g/Hektar und Novaluron mit einer Rate von 30g/Hektar bis 100g/Hektar angewandt.

## Revendications

1. Utilisation d'une composition insecticide, comprenant (a) au moins un composé insecticide ayant une action de choc efficace, sélectionné parmi le groupe (A) composé d'imidacloprid et d'acétamipride, et (b) du novaluron en tant que composé insecticide avec une action à long terme efficace, pour la suppression des insectes dans les cultures et leur emplacement géographique.

2. Utilisation selon la revendication 1, dans laquelle ladite composition insecticide comprend de 0,1 % à 60 % d'au moins un insecticide issu du groupe (A) et de 0,5 % à 60 % de novaluron, de préférence de 10 % à 50 % d'au moins un insecticide issu du groupe (A) et de 5 % à 15 % de novaluron.

3. Utilisation selon la revendication 1, dans laquelle le rapport des poids, entre les insecticides du groupe (A) et le novaluron, est dans la fourchette comprise entre 1:100 et 100:1, de préférence entre 1:50 et 50:1.

4. Utilisation selon l'une quelconque des revendications 1 à 3, dans laquelle ladite composition inserticide contient en outre des excipients et/ou des additifs et/ou des agents tensioactifs.

5. Procédé de suppression des insectes, comprenant l'application d'au moins un composé ayant une action de choc efficace, sélectionné parmi le groupe (A) composé d'imidacloprid et d'acétamipride, et (b) du novaluron en tant que composé insecticide avec une action à long terme efficace, pour la suppression des insectes dans les cultures et leur emplacement géographique

6. Procédé selon la revendication 5, dans lequel l'application des insecticides est simultanée, séparée ou séquentielle.

7. Procédé selon la revendication 5, dans lequel, lorsqu'ils sont appliqués à une culture ou à un champ, les insecticides du groupe (A) sont appliqués à un dosage de 1 g/hectare à 500 g/hectare et le novaluron est appliqué à un dosage de 1 g/hectare à 200 g/hectare, de préférence les insecticides du groupe (A) sont appliqués à un dosage de 100 g/hectare à 350 g/hectare et le novaluron est appliqué à un dosage de 30 g/hectare à 100 g/hectare.
